# EUROPÄISCHE PATENTSCHRIFT

(11) **EP 0 776 644 B1**
(45) Veröffentlichungstag und Bekanntmachung des Hinweises auf die Patenterteilung: **10.10.2001**
(21) Anmeldenummer: 95118756.6
(22) Anmeldetag: 29.11.1995
(51) Int. Cl.: A61F 13/08

(54) **Kompressionsstrumpf zum Zusammenspressen von Krampfadern**
Stocking for compressing varicose veins
Bas de compressions à varices

(43) Veröffentlichungstag der Anmeldung: 04.06.1997
(73) Patentinhaber: Hechmat, Albogh, Dr., 55130 Mainz (DE)
(72) Erfinder: Hechmat, Albogh, Dr., 55130 Mainz (DE)

(56) Entgegenhaltungen:
- DE-A- 3 802 338
- DE-C- 358 246
- US-A- 3 856 008

## Beschreibung

Kompressionsstrumpf zum Zusammenpressen von Krampfadern, bestehend aus einem längsgeschlitzten elastischen Strumpf und einer Mehrzahl von die Längsschlitzränder lösbar und mit variabler Spannkraft miteinander verbindenden, handbetätigbaren Verschlüssen.

Aus der DE OS 42 30 165 ist ein an der Rückseite des Beins längsgeschlitzter Kompressionsstrumpf für menschliche Beine zur Bekämpfung des Varicosesymptomkomplexes bekannt, bei dem der Kompressionsdruck exakt auf eine individuelle Beinanatomie eingestellt werden kann, indem zentimeterweise der therapeutisch erforderliche Druck an einzelnen Beinbereichen reguliert wird.

Mit Klettbändern oder anderweitig verstellbaren Verschlüssen können die Kompressionsstrümpfe an ihren Längsschlitzrändern variabel miteinander verbunden werden, so daß der Druck des Kompressionsstrumpfes der individuell ausgeprägten Krankheit angepaßt werden kann.

Es ist ein Nachteil bei diesem bekannten Kompressionsstrumpf, daß im Knöchelbereich des Fußgelenks die Bewegungsfreiheit durch die zahlreichen Verschlüsse eingeengt werden kann. Ebenfalls nachteilig ist es, daß in Bereichen, in denen der Druck des Kompressionsstrumpfes nicht fein differenziert sein muß, die Anzahl der Verschlüsse auf engem Abstand unnötig groß ist. Außerdem ist die Handhabung der auf der Rückseite des Beins angeordneten Verschlüsse für die Einstellung der verschiedenen Drücke und ihre Verbindung und Lösung eine schwierige durch Sichtbehinderung beeinträchtigte körperliche Anstrengung.

Aus der US-Patentschrift 3,856,008 ist ein Kompressionsstrumpf für das Bein eines Tieres bekannt, der an seiner vorderen Seite zwei Öffnungen aufweist, deren Ränder mittels zahlreicher Klettverschlüsse miteinander verbunden werden können.

Bei Übertragung einer solchen Lösung eines Kompressionsstrumpfes auf menschliche Beine ergäben sich mehrere Schwierigkeiten. Zum einen wäre das Anziehen durch die beiden Öffnungen im Beinvorderbereich sehr erschwert und die schmalen Materialbrükken zwischen den Öffnungen stark beansprucht.

Zudem ist die Zahl der Verschlüsse auf engem Abstand unnötig groß, da in einigen Bereichen des Beines eine Feindifferenzierung des Druckes nicht notwendig ist.

Aufgabe der Erfindung ist es, einen Kompressionsstrumpf für menschliche Beine zu schaffen, der den Bewegungsfreiraum der Sprunggelenke nicht einschränkt und dessen Verschlüsse in der Zahl wesentlich verringert und leichter sowie ohne Sichtbehinderung betätigbar sind.

Gelöst wird diese Aufgabe nach der Erfindung durch einen Kompressionsstrumpf mit den Merkmalen des Anspruchs 1.

Der erfindungsgemäße Kompressionsstrumpf schränkt mit der teilweisen seitlichen Anordnung der Verschlüsse am Bein den Bewegungsfreiraum im Knöchelbereich des Fußgelenks nur noch in geringstmöglicher Weise ein.
Durch die Verwendung wesentliche verbreiterter Klettverschlüsse im Oberschenkelbereich, in dem der Druck weniger differenziert sein muß, wird die Anzahl der Verschlüsse reduziert und der Komfort beim Anlegen des erfindungsgemäßen Kompressionsstrumpfes erhöht.

## Patentansprüche

1. Kompressionsstrumpf zum Zusammenpressen von Krampfadern, bestehend aus einem längsgeschlitzten elastischen Strumpf und einer Mehrzahl von die Längsschlitzränder lösbar und mit variabler Spannkraft miteinander verbindenden, handbetätigbaren Verschlüssen, **dadurch gekennzeichnet, daß**
- die Längsschlitzränder im Knöchelbereich des Fußgelenks auf einer Seite des Fußes
und
- im übrigen Beinbereich auf der Vorderseite des Beins verlaufen,
wobei
- die Verschlüsse im Beinoberbereich bis etwa zehn mal breiter bemessen sind als im Beinunterbereich.

## Claims

1. Compression stocking for compressing varicose veins consisting of an elastic stocking, with longitudinal opening and a number of manually operated closures located on the edges of the longitudinal opening which can be loosened and connected to one another with variable tension and **characterised by** the fact that
- the edges of the longitudinal opening in the area of the ankle joint are on one side of the foot
and
- in the remaining part of the leg are located on the front side of the leg
whereby
- the closures in the upper part of the leg measure up to 10 times the width of those in the lower leg area.

## Revendications

1. Bas de compression à varices, consistant en un bas élastique fendu dans la longueur et en plusieurs fermetures manuelles servant à relier les bords de la fente longitudinale l'un à l'autre avec une tension variable et permettant de les détacher; **caractérisé par** le fait que
- au niveau de la cheville, les bords de la fente longitudinale se trouvent sur un côté du pied
et
- sur le reste de la jambe, ils se trouvent sur le devant de la jambe
- les fermetures situées au niveau de la cuisse étant jusqu'à environ dix fois plus larges qu'au niveau de la partie inférieure de la jambe.
